# EUROPEAN PATENT SPECIFICATION

(11) **EP 4 424 184 B1**
(45) Date of publication and mention of the grant of the patent: **01.10.2025**
(21) Application number: 24188996.3
(22) Date of filing: 26.10.2018
(51) Int. Cl.: A24F 40/46, A24F 40/57

(54) **LOW-TEMPERATURE VAPORIZER AND LOW-TEMPERATURE SMOKING SET**
NIEDERTEMPERATURVERDAMPFER UND NIEDERTEMPERATURRAUCHSET
VAPORISATEUR À BASSE TEMPÉRATURE ET ENSEMBLE À FUMER À BASSE TEMPÉRATURE

(30) Priority: 27.10.2017 CN 201721416144 U; 12.10.2018 CN 201811193550
(43) Date of publication of application: 04.09.2024
(62) Divisional of application: 18202994.2
(73) Proprietor: Shenzhen First Union Technology Co., Ltd., 518103 Shenzhen, Guangdong (CN)
(72) Inventor: WU, Zexin, Shenzhen, 518103 (CN); LI, Yonghai, Shenzhen, 518103 (CN); XU, Zhongli, Shenzhen, 518103 (CN)
(74) Representative: Proi World Intellectual Property GmbH

(56) References cited:
- WO-A1-2011/109849
- CN-A- 104 522 891
- US-A1- 2011 155 153
- US-A1- 2015 007 835

## Description

### TECHNICAL FIELD

The present disclosure relates to the field of low-temperature baked smoking sets, and particularly, to a low-temperature baked vaporizer and a low-temperature baked smoking set having same.

### BACKGROUND ART

The low-temperature baked smoking sets mainly use some solid vaporizable materials such as tobacco shreds or opium paste etc. to be baked at a low-temperature to generate smoking smog for inhaling. For this low-temperature baked smoking sets, their structure always has a hollow cylindrical vaporizer. In use, the solid vaporizable materials are disposed inside the cylindrical vaporizer, by the vaporizer, the solid vaporizable materials are heated to generate smoking smog. Such a smoking set is known from US-A-2015/0007835 or CN-A-104522891.

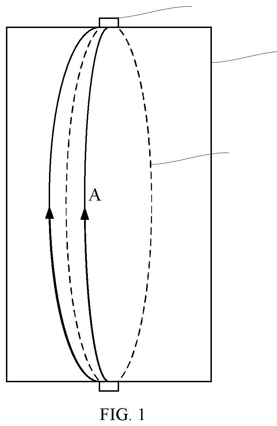

In practice, to match up with the cylindrical shape of the solid vaporizable materials, the vaporizer is shaped like a cylinder. To let the vaporizer work well, normally electrode pins are welded on two ends of the vaporizer. In the process that the vaporizer is in use, the main shortage is uneven heating of the cylindrical vaporizer, which contributes to unevenly generated smoking smog. More specifically, the unfolded the cylindrical vaporizer shows the heating area and the heating element 1 in FIG. 1. Two longitudinal ends of the heating element 1 both have an electrode pin 2 for connecting the power supply. After giving the electricity, even though the heating element 1 as a whole is manufactured by the conductive materials, the current itself prefers the shortest itinerary with minimum resistance to form a current loop. For example, the two current loops A and B are shown in FIG. 1, the current loop A has a shorter itinerary than the current loop B between the two electrode pins 2, then most current prefers the current loop A to form the current loop, so most heat generated by the heating element 1 is centralized on the main heating area 3 in FIG. 1, whereas, the remaining area has less heat, so the heating element 1 as a whole generates heat unevenly.

### SUMMARY

In view of the drawbacks in the prior art that vaporizable materials are heated unevenly by a heating element, the present disclosure relates to a low-temperature baked vaporizer.

In order to solve the above technical problem, the present invention provides a low-temperature baked vaporizer according to independent claim 1.

Preferably, the heating element has a cut, configured for the heating element to be easily sleeved on the sleeve; the cut is axially bored on the heating element, through a side wall of the heating element.

Preferably, the heating element includes at least one heating area extending along an axial direction thereof, and each heating area has electrode connecting parts.

The present invention further provides a low-temperature baked smoking system, according to claim 4, having the aforementioned low-temperature baked vaporizer. The smoking system includes the aforementioned low-temperature baked vaporizer; and a power supply configured for supplying power to the vaporizer.

Additional aspects and advantages of the present disclosure will be: the vaporizer and the electronic cigarette having the same bake the solid vaporizable materials to generate smoking smog, unlike traditional smoking sets which need to burn the vaporizable materials, so a variety of carcinogens are avoided when the vaporizable materials are burned, to decrease the damage to users. Moreover, compared with the traditional electronic cigarettes that the tobacco liquid is aerosolized, the smoking taste of the vaporizer and the electronic cigarette is more pure.

### BRIEF DESCRIPTION OF THE DRAWINGS

Many aspects of the present disclosure can be better understood with reference to the following drawings. The components in the drawings are not necessarily drawn to scale, the emphasis instead being placed upon clearly illustrating the principles of the present disclosure. Moreover, in the drawings, like reference numerals designate corresponding parts throughout the several views.
FIG. 1 illustrates an unfolded heating element of a low-temperature baked smoking set in the prior art;
FIG. 2 is an isometric view of the low-temperature baked smoking set in accordance with an embodiment of the present disclosure;
FIG. 3 is an isometric view of a vaporizer in the low-temperature baked vaporizer in accordance with an embodiment of the present disclosure;
FIG. 4 illustrates the heating element of FIG. 2;
FIG. 5 illustrates the power supply module of FIG. 2;
FIG. 6 illustrates the assembled vaporizer in the low-temperature baked smoking set.
FIG. 7 illustrates the sleeve of FIG. 6.
FIG. 8 illustrates the heating element of FIG. 6;
FIG. 9 illustrates the heating element unfolded along a circumferential direction thereof in accordance with another embodiment of the present disclosure;
FIG. 10 illustrates the heating element unfolded along a circumferential direction thereof in accordance with another embodiment of the present disclosure;
FIG. 11 illustrates the heating element unfolded along a circumferential direction thereof in accordance with another embodiment of the present disclosure;
FIG. 12 illustrates the heating element unfolded along a circumferential direction thereof in accordance with the present invention;
FIG. 13 illustrates the heating element unfolded along a circumferential direction thereof in accordance with the present invention;
FIG. 14 illustrates the heating element unfolded along a circumferential direction thereof of FIG. 8;
FIG. 15 illustrates the heating element unfolded along a circumferential direction thereof in accordance with another embodiment of the present disclosure;
FIG. 16 illustrates the heating element unfolded along a circumferential direction thereof in accordance with another embodiment of the present disclosure;
FIG. 17 illustrates the heating element unfolded along a circumferential direction thereof in accordance with another embodiment of the present disclosure;
FIG. 18 illustrates the low-temperature baked smoking set assembled with the solid vaporizable materials in accordance with an embodiment of the present disclosure.

### DETAILED DESCRIPTION

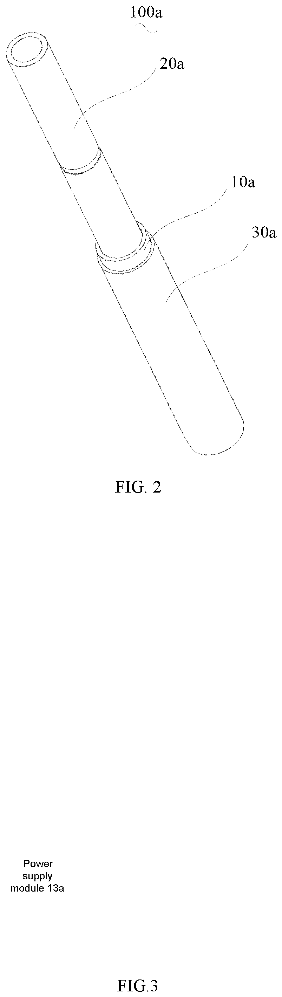

The structure and operating principle of the above low-temperature baked vaporizer and the low-temperature baked smoking set are illustrated below, mainly shown from FIG. 2 to FIG. 4 in further detail using exemplary embodiments.

Referring to FIG. 2, which is an isometric view of the low-temperature baked smoking set in accordance with an embodiment of the present disclosure. The low-temperature baked smoking set 100a includes a low-temperature baked vaporizer 10a and a housing 30a. The vaporizer 10a is accommodated inside the housing 30a. The vaporizer 10a is configured for receiving a cartridge 20a that is heated to generate smoking smog.

Referring to FIG. 3, in this embodiment, the vaporizer 10a includes a sleeve 11a, a heating element 12a and a power supply module 13a. The sleeve 11a is configured for receiving vaporizable materials, that is tobacco cigarette; a heating element 12a, sleeved outside the sleeve 11a, and configured for heating the sleeve 11a; the power supply module 13a is electrically connected with the heating element 12a to heat the heating element 12a.

The sleeve 11a is roughly round, made by metal materials, at least any one selected from a group of pure metals, alloys, metallic compounds or special metals etc. such as iron, copper, aluminum, tin, nickel, gold, silver, lead, zinc or other alloys. An insulating layer (not shown) is disposed outside the sleeve 11a and configured for avoiding the sleeve 11a to be thermal conducted with the heating element 12a. The insulating layer is made up with insulating materials, at least any one selected from a group of synthetic resin, epoxy resin, phenolic resin, 4250 silicone plastic asbestos and polyimide plastic etc. The sleeve 11a has a receiving chamber 110a configured for receiving the solid vaporizable materials. An inner diameter of the receiving chamber 110 is defined as 5~8mm, to ensure the solid vaporizable material to be easily inserted into and let the solid vaporizable material tightly abut against the receiving chamber, therefore, improving efficiency of heating the solid vaporizable material.

In some embodiments, the insulating layer sleeved outside the sleeve 11a is an oxide layer, for example, to oxidate outside surface of the sleeve 11a forms the oxide layer. by relying on the oxide layer, it avoids the sleeve 11a to be thermal conducted with the heating element 12a.

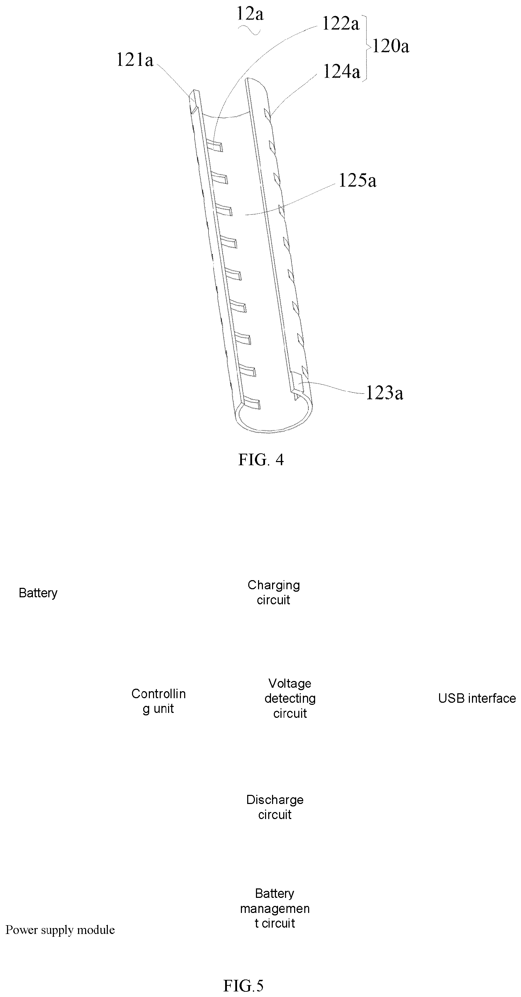

Further referring FIG. 4, the above heating element 12a is shaped like a hollow cylinder, made up with metallic materials, such as stainless steel sheets in this embodiment. The stainless steel sheet brings the heating element 12a fashionable and beautiful appearance, strong corrosion resistance that prolongs using life of the vaporizer 10a. In some embodiments, a thickness of the heating element 12a is 0.4mm that may accelerate the heating speed.

The heating element 12a includes a first electrode 121a and a second electrode 123a; the first electrode 121a and the second electrode 123a are electronically connected with the power supply module 13a to heat the heating element 12a. As shown in FIG. 4, the first electrode 121a and the second electrode 123a are respectively disposed at two longitudinal ends of the heating element 12a to make the current through the heating element 12a completely.

The heating element 12a has numerous sets of through holes 120a, divided into first sets of through holes 122a and second sets of through holes 124a; the first sets of through holes 122a and second sets of through holes 124a are nearly strip-shaped holes, configured for adjusting resistance of the heating element 12a. The first sets of through holes 122a and second sets of through holes 124a are symmetrically set with each other, to make the current evenly through the heating element 12a. Each set of through holes122a and 124a includes at least one through holes that is axially dispersed on the heating element 12a. The number of sets of first through holes 122a and the number of sets second through holes 124a may be determined based on different situations. More specifically, the first sets of through holes 122a and second sets of through holes 124a are symmetrically set with each other, to make the current evenly through the heating element 12a, so the heating element 12a heats evenly.

In other embodiments, there are no sets of through holes 120a, whereas, by diminishing or increasing the length of the heating element 12a, it may adjust resistance of the heating element 12a.

In other embodiments, the heating element 12a has a cut 125a, configured for the heating element 12a to be easily sleeved on the sleeve 11a; the cut 125a is axially bored on the heating element 12a, through a side wall of the heating element 12a. The first electrode 121a and the second electrode 123a may be disposed at two sides of the cut 125a, so the heating element 12a heats completely.

Referring to FIG. 5, the above power supply module 13a may be respectively connected with the first electrode 121a and the second electrode 123a via threads, to be electrically connected with the heating element 12a. The power supply module 13a includes an USB interface 130a, a battery 131a, a control unit 132a, a charge circuit 133a, a discharge circuit 134a, a voltage detecting circuit 135a, two switches 136a and a battery management circuit 137a. The battery 131a is respectively connected with the charge circuit 133a and the discharge circuit 134a. Two switches 136a are respectively disposed between the battery 131a and the charge circuit 133a, and between the battery 131a and discharge circuit 134a. The charge circuit 133a and the discharge circuit 134a are both electrically connected with the USB interface 130a, the discharge circuit 134a is electrically connected with the battery management circuit 137a; the battery management circuit 137a is electrically connected with the heating element 12a; the voltage detecting circuit 135a is electrically connected with the USB interface 130a; the control unit 132a is connected with the two switches 136a and the voltage detecting circuit 135a respectively. The discharge circuit 134a is electrically connected with the battery management module 137a. The battery management module 137a is configured for supplying power to the heating element 12a. If the voltage detecting circuit 135a receives the voltage, that means, the power supply module 13a is connected with the external power supply, the voltage detecting circuit 135 sends an electricity signal to the control unit 132a; after the control unit 132a receives the electrical signal, the control unit 132a controls the switch 136a between the battery 131a and charging circuit 133a to make "off" state alternative to "on" state, so the current from the external power supply supplies power to the battery 131a through the charging circuit 133a. If the voltage detecting circuit 135a fails to detect the voltage, that means the power supply module 13a fails to be electrically conducted with the external power supply, the control unit 132a generates another electrical signal, the control unit 132a receives the electrical signal and controls the other switch 136a between the battery 131a and the discharge circuit 134a, making "off" state alternative to "on" state, the current passes towards the heating element 12a through the discharge circuit 134a and the battery management module 137a.

In this embodiment, the vaporizer 10a includes the sleeve 11a, the heating element 12a and power supply module 13a. By relying on electrical connection between the power supply module 13a and the heating element 12a, the heating element 12a is sleeved outside the sleeve 11a. The heating element 12a has a plurality sets of through holes 120a, for adjusting the resistance of the heating element 12a that can evenly heat the whole sleeve 11a. The sleeve 11a and the heating element 12a made by metallic materials, which can improve the heating temperature, make the heating element 12a produce smoking smog faster and eventually improve the user experience.

In these embodiments, the solid vaporizable materials 20a may be at least one or more selected from a group of tobacco slices and tobacco sauces, or a group of tobacco rods, tobacco paste or herbs etc.

In these embodiments, the housing 30a is nearly a hollow cylinder, configured for receiving the vaporizer 10a. The housing 30a may be a plastic shell such as polycarbonate, polyurethane, polyimide and some plastic materials with good heat preservation effect. In some embodiments, the housing 30a is made from a metallic housing with coating a plastic membrane to itself, which comes to effect of heat preservation.

In these embodiments, the low-temperature baked smoking set 100a includes a vaporizer 10a for improving the heating temperature, making the low-temperature baked smoking set 100a available for producing smoking smog faster, therefore the user experience is effectively improved.

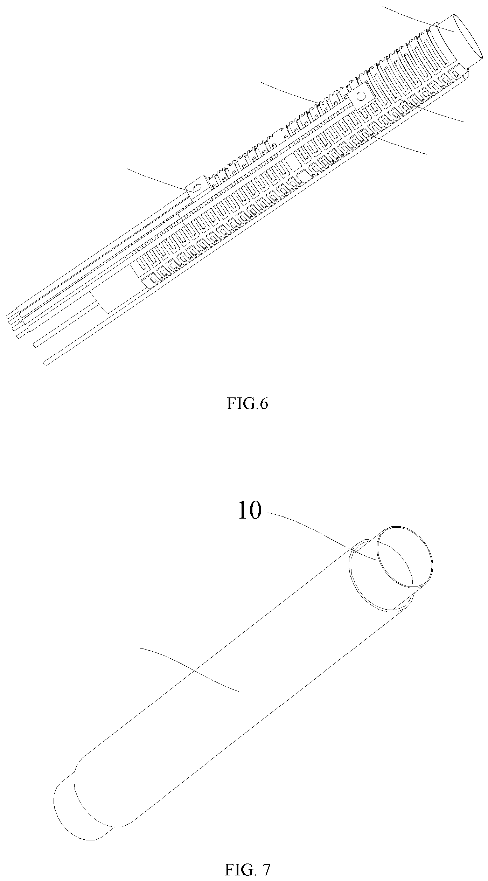

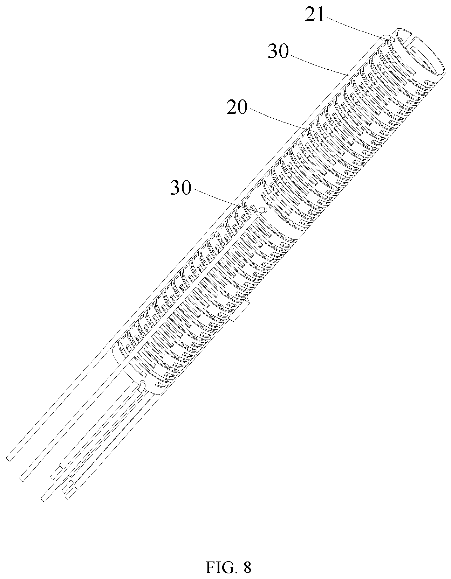

Beyond the above embodiments, the present disclosure relates to another vaporizer in accordance with another embodiment, as shown from FIG. 6 to FIG. 8. FIG. 6 illustrates the assembled vaporizer in the low-temperature baked smoking set. FIG. 7 illustrates the sleeve of FIG. 6. FIG. 8 illustrates the heating element of FIG. 6. The vaporizer include a hollow sleeve 10, a heating element 20 surrounded around the hollow sleeve 10 and an electrode connector 30 carried on the heating element 20.

The sleeve 10 is made from thermal conductive materials, configured for receiving the solid vaporizable materials inside;

The shape of the heating element 20 is matched with the cylindrical-shaped of the sleeve 10, since the heating element 20 is sleeved outside the sleeve 10, configured for heating the vaporizable materials.

The electrode connector 30 is defined as an electrode pin as shown in FIG. 6 to FIG. 8, and configured for connecting with an output electrode of the power supply module, supplying power to the heating element 20 for generating heat. It makes sense that the electrode pins are the most common electrode connecting components in the prior arts; except for the electrode pins in the present disclosure, the electrode connectors 30 further may be electrode poles or terminals etc.

Furthermore, in accordance with the above embodiments, the heating element 20 is made from electric materials in favor of heating the vaporizable materials, such as pure nickel alloys, nickel-chrome alloys, nickel-iron alloys, iron-chromium alloys, iron-chromium-aluminum alloys, titanium alloys and stainless steels etc. But the sleeve 10 is made from better thermal-conductive materials, like metallic materials, such as pure metals, alloys, metallic compounds or specialty materials etc., of which the alloys are composed of at least one of irons, coppers, aluminums, tins, gold, silver etc. Since the sleeve 10 adopts the metallic materials, electrical-conduction between the heating element 20 and the sleeve10 is avoided, by an insulating layer 40 disposed between the sleeve10 and outside surface of the heating element 20. The insulating layer 40 is made by methods of sputtering, deposition, coating, or attaching films to the surface of the sleeve 10. For a purpose of insulation, the insulating layer 40 itself is made from at least one selected from a group of synthetic resins, polyimide resins, polyurethane resins and metallic oxides etc. The sleeve 10 is configured for receiving the vaporizable materials, inner diameter of the sleeve 10 has to be matched up with the diameter of the vaporizable materials, 5~8mm are chosen in the present invention, which ensures smooth insertion of the vaporizable materials and ensures the vaporizable materials to tightly abuts against the sleeve 10, eventually improving the heating efficiency.

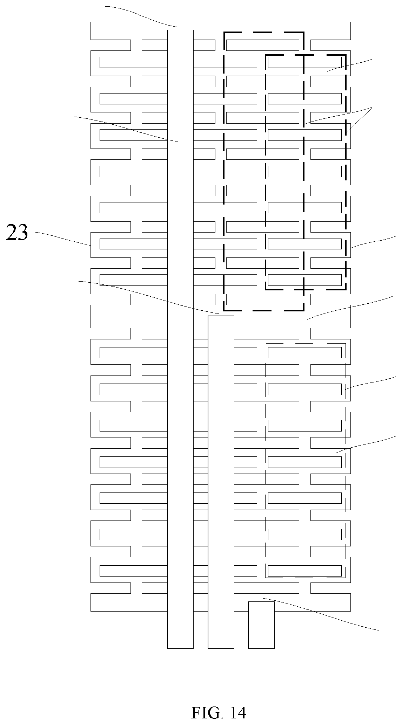

Furthermore, the cylindrical-shaped heating element 20 is formed by wrapping a layer-shaped object around the sleeve 10 along its width direction. To more clearly see the shape of the heating element 20 itself and structure, FIG. 8 illustrates a heating element 20 in use according to embodiments of the present disclosure. FIG. 14 also illustrates the heating element unfolded circumferentially of FIG. 8. The heating element 20 itself as a layer-shaped structure is wrapped around the outside surface of the sleeve 10 which forms a cylinder to match up with the sleeve 10. The layer-shaped heating element 20 has an even thickness from a range of 0.03~0.1mm.

Further referring to FIG. 8 to FIG. 14, two longitudinal ends of the heating element 20 include the electrode connecting parts 21, the above electrode connectors 30 are electrically connected with the electrode connecting parts 21. Of course, the number of the electrode connecting parts 21 is at least two. For example, the first electrode connecting part 211 and the second electrode connecting part 212 are respectively connected with a positive electrode and a negative electrode of the power supply to from current loops. The material of the electrode connecting parts 21 is the same as the electrode connector 30. In embodiments of the present disclosure, the electrode connectors 30 are electrode pins, then the corresponding electrode connecting parts 21 are pin welding points. In some embodiments, the electrode connectors 30 are plug terminals, the electrode connecting parts 21 are plug points available for the plug terminals. Under the circumstance, based on the shape of the heating element 20, it is best to adopt the way of pin welding. Of course, in other feasible situations, multiple other welding methods may be adopted.

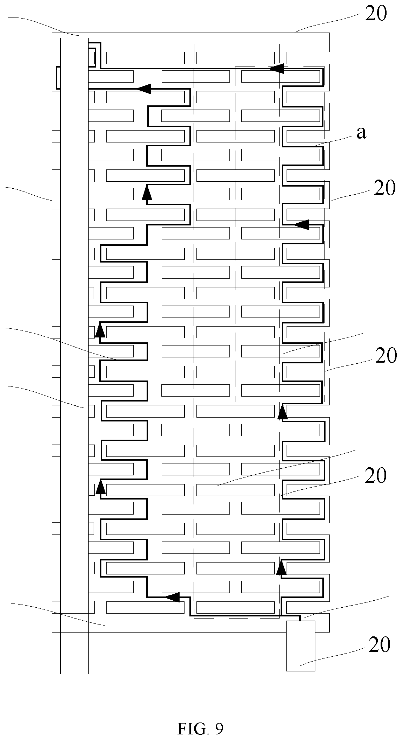

Furthermore, as shown in FIG. 6, and from FIG. 9 to FIG. 13, the above heating element 20 has a first side edge 23 and a second side edge 24 along a breadth direction thereof. The above first electrode connecting part 211 and second electrode connecting part 212 both are disposed at a certain position, like, a central position between the first side edge 23 and the second side edge 24. As shown in FIG. 6, when the heating element 20 is wrapped around the sleeve 10, the first side edge 23 and the second side edge 24 are closing but contactless with each other, remaining about 1~ 5mm distance therebetween, which may basically cover the circumferential surface of the sleeve 10, but also avoid overheating due to the contact of the first side edge 23 and the second side edge 24.

Furthermore, to make the current evenly passing through the heating element 20, and ensure every area of the heating element 20 with a suitable resistance, the heating element 20 is bored with some through holes. For example, according to the power for heating in normal use, the whole resistance of the heating element 20 maintains at 04-1.0 ohms. As shown from FIG. 9 to FIG. 13, the heating element 20 is opened with a plurality sets of through holes 22 along a breadth direction or a circumferential direction of the heating element 20. The plurality sets of through holes 22 are provided to increase resistance of an area of the heating element 20 near to the electrode connecting part 21 and also increase resistance of an area of the heating element 20 far away from the electrode connecting part 21, as an aid to equal the resistances of the area near to the electrode connecting part 21 and the area far away from the electrode connecting part 21, so the whole heating element 20 circumferentially generates heat evenly. For achieve an even resistance, the plurality sets of through holes 22 are arrayed unevenly, the array of sets of through holes is shown from FIG. 9 to FIG. 13.

**In** terms of the heating element 20 in FIG. 9, two longitudinal ends of the heating element 20 are the electrode connecting parts 21, i.e. the first electrode connecting part 211 and the second electrode connecting part 212, which are respectively connected with the positive and negative terminals of the power supply via the electrode connectors 30. Each set of through holes 22 arrayed on the heating element 20 has at least one through hole 221 along a length direction, that is, an axial direction after the heating element 20 is wrapped. Along a breadth direction, adjacent sets of through holes 22 are staggered with each other, for example, the adjacent set of through holes 22A and the adjacent set of through holes 22B are arrayed in the staggered manner, not in the aligned manner, therefore, the current path and resistance would be more diffusive, to let the heating element 20 generate heat more evenly. For example, in FIG. 9, two currents a and b are dispersed at two different areas, but the current density, current value and resistance of currents a and b come to the same, therefore, the heat generated by the heating element 20 is basically the same in different areas.

According to the embodiment shown in FIG. 9, the through holes221 belonging to one same set of through holes 22 may be same in the shape, the size and the distance. Of course, the through holes 221 belonging to the same set of through holes 22 may be different in the shape, the size and the distance.

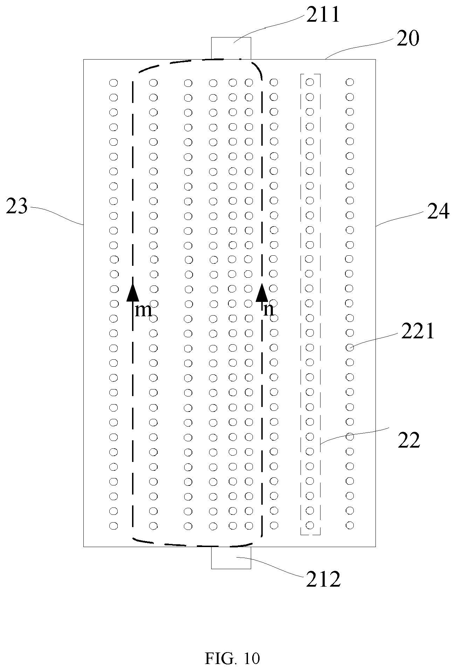

As shown in FIG. 10, which illustrates the heating element unfolded along a circumferential direction thereof in accordance with another embodiment of the present disclosure. **In** this embodiment, each set of through holes 22 has at least one through holes 221 along a length direction of the heating element 20. When these sets of through holes 22 are arrayed along a breadth direction of the heating element 20, the distance between adjacent sets of through holes 22 gets larger along a direction deviating from the electrode connecting part 21, basically, to make smaller size of through holes closing to the first side edge 23 or the second side edge 24 compared with through holes closing to the electrode connecting part 211. **In** this case, the resistances of different areas in the heating element 20 have been changed again, and the distance between adjacent sets of through holes 22 gets larger along a direction deviating from the electrode connecting part 21, so the resistance of an area closing to the electrode connecting part 21 is increasing, and the resistance increase rate of the area near to the electrode connecting part 21 is larger than the resistance increase rate of the area far away from the electrode connecting part 21,as an aid to adjust the resistance of the whole heating element 20 with consequently even current, so the whole heating element 20 circumferentially generates heat evenly. For example, FIG. 10 shows two current paths m and n, even through the current path m has longer itinerary then the current path n from the electrode connecting part 211 to the electrode connecting part 212, since the adjusted resistances of the current path m and the current path n come to be same, finally, the current paths m and n have equal current value, therefore generating even heat.

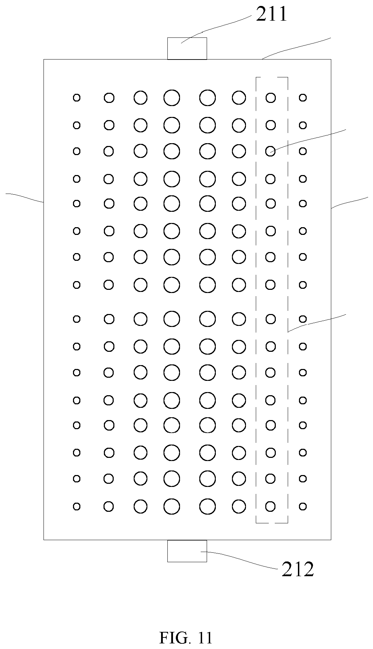

Meanwhile, other embodiments of the present disclosure relate to another method to achieve even resistance of the whole heating element 20 by designing sets of through holes 22 arrayed along a breadth direction. As shown in FIG. 11, the adjacent sets of through holes 22 have the same distance along a breadth direction, but with deviating from the electrode connecting part 21 the size of each through hole 221 in each set of through holes 22 is decreasing, under the circumstance, the resistance of the area closing to the electrode connecting part 21 is increasing, the resistance increasing rate of the area near to the electrode connecting part 21 is larger than a resistance increasing rate of the area far away from the electrode connecting part 21, as an aid to even the resistance of the whole heating element 20, consequently, the heating element 20 generates even heat. Likewise, the through holes 221 in one set of through holes 22 have same size and are arrayed in same distance between adjacent through holes 221 along the length direction thereof, which contributes to even the resistance of the heating element 20 along the length direction thereof.

Understandable, the above two arraying method in the aforementioned embodiments in FIG. 10 and FIG. 11 may be combined in use, the sets of through holes 22 may be arrayed with that the sizes are gradually deceasing and the distances are gradually increasing simultaneously along a breadth direction of the heating element 20, as another aid to even the resistance of the whole heating element 20.

Understandable, the embodiments as shown in FIG. 10 and FIG. 11, the through holes 221 in the sets of through holes 22 have variable sizes and distances along a direction deviating from the electrode connecting part 21. Since the electrode connecting parts 21 are respectively disposed at middle of two longitudinal ends of the heating element 20, to make the main current paths as shown in FIG. 10 and FIG. 11 distributive around the shortest itinerary from the upper and lower electrode connecting parts 21. Therefore, by relying on the electrode connecting parts 21 as a start of the variable through holes 221 to change the sizes and distances of the through holes 221, these variable through holes 221 may make the resistances of different areas of the heating element 20 even. When the electrode connecting parts 21 are not at middle as shown in FIG. 10 and FIG. 11, the through holes 221 in the sets of through holes 22 with variable sizes, distances and the start of the variable through holes 221 have to be in accordance with the position of the electrode connecting parts 21.

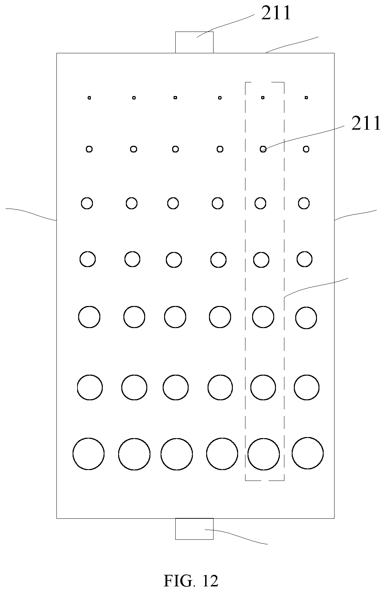

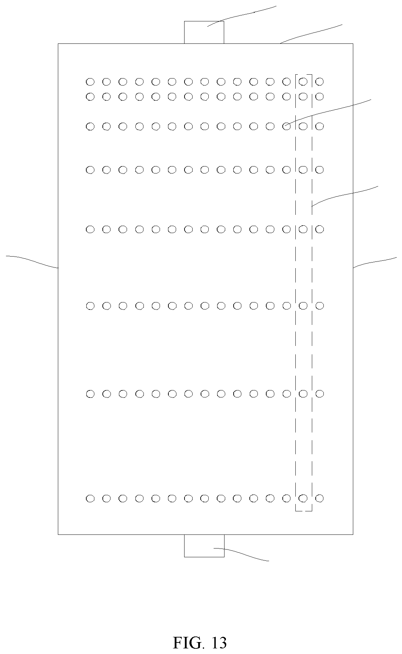

Further, referring to FIG. 12 and FIG. 13, FIG. 12 illustrates the heating element unfolded along a circumferential direction thereof in accordance with the present invention. Under the circumstance, through holes 221 in each set of through holes 22 are arrayed with decreasing sizes along the length direction. However, in FIG. 13, which illustrates the heating element unfolded along a circumferential direction thereof in accordance with the present invention. All through holes 221 in each set of through holes 22 are arrayed with increasing distances along the length direction.

With the aforementioned two kinds of arrays of through holes 221 in sets of through holes 22in changing sizes and distances, because of the current paths dividing and combining manifolds along the length direction, the resistance of the whole heating element 20 has changed, that means the heating area on the original heating element 20has been decentralized and restrained, so as to even the resistance of the heating areas on the heating element 20.

**In** some embodiments, the through holes 221 in sets of through holes 22 may have variety of shapes, such as round, rectangular or hexagon and so on.

During the low-temperature smoking set is inhaled, since at beginning the vaporizable materials, like a brand new tobacco cigarette has a plenty of tobacco, a big amount of smoking smog may be generated, but with the increasing time for baking the tobacco cigarette, the amount of smoking smog will be decreased. So after the tobacco cigarette is baked for a while, to improve the amount of smoking smog and even the amount of smoking smog in a whole process of inhaling, a heating element 20 as shown in FIG. 14 is adapted. In the embodiment, the heating element 20 has two heating area in serial connection along a length direction thereof, that is, a first heating area 210 and a second heating area 220, which are both disposed between the first side edge 23 and the second side edge 24. The first and second heating areas 210, 220 are in serial connection via a connector 230. The first and second heating areas 210, 220 are both opened with sets of through holes 22, nonetheless, the connector 230 is a region with no through holes. By relying on the first heating area 210 and a second heating area 220 in serial connection, they may adjust the length of the tobacco cigarette to improve the amount of smoking smog. More specifically, the longitudinal end of the first heating area 210 has a first connecting part 211, an opposite end of the second heating area 220 has a second connecting part 213. And the connector 230 in serial connection with the first heating area 210 and the second heating area 220 has a connecting part 212 shared by the first heating area 210 and the second heating area 220. The first heating area 210 and the second heating area 220 are respectively electrically connected with the power supply via the first connecting part 211, the second connecting part 213 and the shared connecting part 212. During an initial heating process, the amount of the smoking smog when baking the tobacco cigarette is quite large, it is available to let the first electrode connecting part 212 and the shared connecting part 212 be electrically connected with the positive and negative ends of the power supply via the electrode connectors 30, that are the electrode pins, so that the first heating area 210 works to heat the tobacco cigarette. As the heating time extends, the amount of the smoking smog is gradually reducing, so it needs to change electrode connecting methods: electrically connecting the first electrode connecting part 212 and the second electrode connecting part 213 respectively with positive and negative terminals of the power supply, therefore, the first heating area 210 and the second heating area 220 are both at the heating status then the heating zone and the amount of the smoking smog will be improved. Under the circumstances, the above segmented heating method is used to heat multiple heating areas of the heating element 20, and the control method for controlling the heating process in different statuses, the smoking smog come to be even during different statuses of heating the tobacco cigarette.

Of course, in the above embodiments of segmented heating, the first heating area 210 works first, then the first heating area 210 and the second heating area 220 work simultaneously. In another embodiment, the second electrode connecting part 213 and the shared electrode connecting part 212 are firstly connected with positive and negative terminals of the power supply to let the second heating area 220 work firstly, until the smoking smog reduces then replaced by that the first electrode connecting part 211 and the second electrode connecting part 213 are respectively connected with positive and negative terminals of the power supply, in this case, the first heating area 210 and the second heating area 220 both works to improve the smoking smog.

In the above embodiment as shown in FIG. 14, the number of the heating areas are two, however, in some embodiments, the number is there, four or more, with correspondingly more electrode connecting parts needed. The segmented heating method may let partial heating area of the heating element work firstly, after a while, much more heating areas thereof work. The specific number of heating areas may be determined by the length of the tobacco cigarette and each heating area. When being controlled, the electrode connecting parts corresponding to the heating areas may be electrically connected.

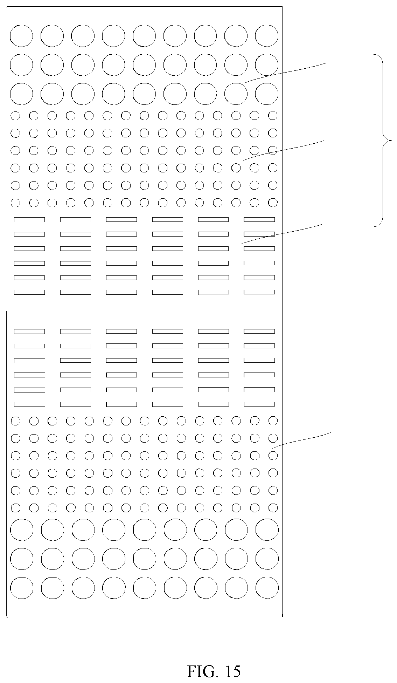

Based on the above embodiment in FIG. 14, the heating element 20 includes multiple heating areas, such as the first heating area 210 and the second heating area 220. Also, another embodiment of the present disclosure relates to another method to design the heating element 20, each heating area has multiple heating zones. The first heating area 210 includes three heating zones, that is a first heating zone 2110, a second heating zone 2120 and a third heating zone 2130. Each heating zone has several through holes in array-arrangement. The through holes in different heating zones have different shapes and/or sizes, compared with other through holes in other heating zones, like the three heating zones in FIG. 15, through holes with different sizes and shapes are arranged along the length direction, such as comparatively large holes, small holes and square-shaped holes, to further make the resistance of each heating zone come to be the same, so as to improve evenness of heating. Likewise, the second heating area 220 is similar with the first heating area 210, which includes three different through holes with different sizes and shapes. In some embodiments, the number of the heating zones is four or more except from three heating zones in the embodiment, each heating zone has different dimensioned and/or shaped through holes, such as hexagonal or diamond-shaped through holes.

From the aforementioned embodiments, the first heating area 210 includes three heating zones along the length or longitudinal direction of the heating element 20. In some embodiments, the heating zones are arranged along the breadth direction or horizontal direction of the heating element 20.

Based on the sectional heating method, similar to FIG. 14, it makes sense that the longitudinal end of the first heating area 210, the opposite end of the first heating area 210 and the second heating area 220, the serial connected area between the first heating area 210 and the second heating area 220 of the heating element 20 have the electrode connecting parts respectively, for connecting with the positive and negative terminals of the power supply, therefore sectional heating of the tobacco cigarette is realized to make the smoking smog even.

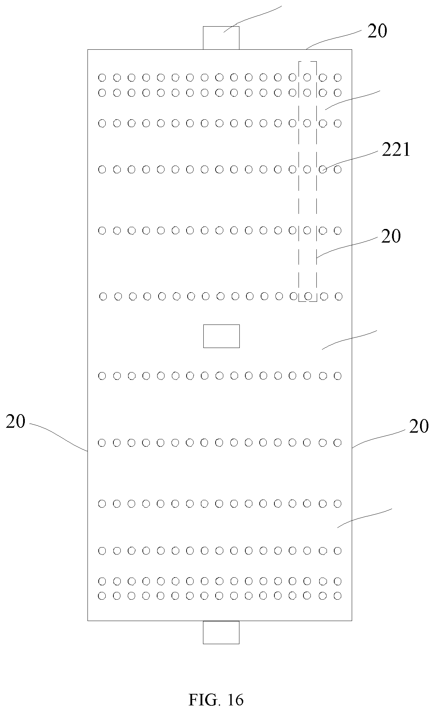

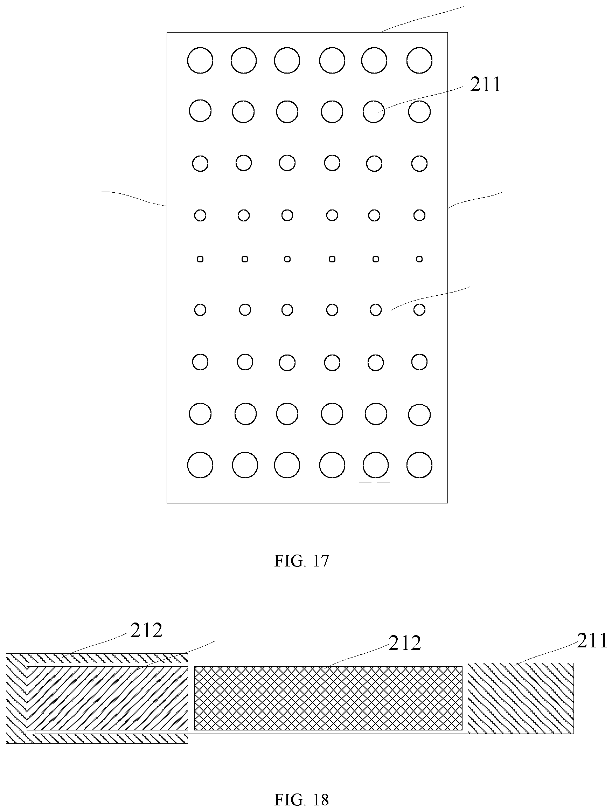

Likewise, based on the above sectional heating method on the heating element 20, another design of the heating element 20 is shown in FIG. 16 and FIG. 17, and referring to FIG. 16, the heating element 20 also includes two heating areas 210 along a length direction. Each heating area 210 has sets of through holes 22 along a breadth direction of the heating element 20, each set of through holes 22 includes several through holes 221 along the length direction. Meanwhile, through holes 22 in each set of through holes are dimensioned with gradually increasing sizes. The longitudinal end, the opposite end, and between two heating areas 210 respectively have the electrode connecting parts 21 for electrically connecting the power supply. The sectional heating method may be the same as the above embodiment, firstly one heating area 210 works to generate heat by electrically connecting corresponding electrode connecting parts 21, after a while, the whole heating element 20 works to generate heat by electrically connecting the longitudinal end and opposite end of the whole heating element 20, the controlling method realizes the sectional heating.

According to the embodiment in FIG. 17, the controlling method for controlling the heating element 20 to work is similar to FIG. 16, two heating areas are configured for sectional heating, that is the first heating area 210 and the second heating area 220; the differential is that the through holes 221 in each set of through holes 22 are dimensioned with gradually decreasing sizes. In terms of segmental heating, the first heating area 210 has same sets of through holes 22 with the second heating area 220 in same size and same array, such that the first heating area 210 has a same resistance with the second heating area 220. Or the first heating area 210 has different sets of through holes 22 with the second heating area 220 in different size and different array, just to guarantee that the first heating area 210 has a same resistance with the second heating area 220.

In view of the above, the heating element 20 may work as a whole to heat simultaneously, or as the sectional heating in accordance with the aforementioned embodiments. At least two heating areas are arrayed along the axial direction. And each heating area owes corresponding electrode connecting parts for electrically connecting with the power supply, controlled independently. Part of the heating areas is chosen to work when demanded, to realize the effect of sectional heating the tobacco cigarette.

With the above arrays of sets of through holes in changing sizes, changing distances or staggered with each other, the resistance of whole heating element has changed, and comes to even. Meanwhile, referring to the three heating zones in the embodiment of FIG. 15, one heating area is divided into at least two heating zones, different heating zone has different sets of through holes in different shapes and sizes, the resistance of whole heating element has changed too, therefore realizing even heating of the heating element 20.

In the above embodiments, by changing the sets of through holes in the heating element 20, the resistance of the heating element has reduced longitudinally, to assemble and contact with the tobacco cigarette. More specifically, for low-temperature baked smoking set, the tobacco cigarette has to be inserted into the sleeve of the vaporizer, then inhaled. FIG. 18 illustrates the low-temperature baked smoking set assembled with the tobacco cigarette. The tobacco cigarette includes a mouthpiece 100, a cooling filler 200 and tobacco segments 300 disposed along the axial direction. In use, the tobacco segments 300 are inserted into the vaporizer 400 for being baked, which generates around 260 degree smoking smog, then smoking smog will be cooled by the cooling filler 200, eventually inhaled via the mouthpiece 100. The cooling filler 200 usually has polymer materials for cooling the smoking smog, avoiding overheated smoking smog to be inhaled to scald users. Therefore, when inserting the tobacco cigarette into the vaporizer 400, a certain distance between the vaporizer 400 and the cooling filler 200 is remained, instead of the vaporizer 400 contacting the polymer materials in the cooling filler 200 to burn the polymer materials or produce noxious substances. If the low-temperature baked smoking set adopts the aforementioned vaporizer 400 as shown from FIG. 9 to FIG. 18, the through holes 221 are arranged on the heating element 20 to change the heating areas, the heating areas will be centralized in those areas bored with the through holes 221 while less heat will be generated at the two longitudinal opposite ends of the heating element 20, even through the longitudinal ends of the heating element 20 contacts the cooling filler 200, the temperature is not high enough to burn the polymer materials or let the polymer materials produce noxious substances. So in the process of inserting the tobacco cigarette into the vaporizer 400, the tobacco cigarette may be deeply inserted to abut against the bottom of sleeve of the vaporizer 400, no need for a distance therebetween, which is more convenient.

To monitor the heating process of the heating element itself, based on the above embodiments, a temperature sensor 50 is mounted on the heating element 20, as shown in FIG. 6, according to structure characters of the heating element 20 itself, the temperature sensor 50 may be inserted into the through holes to keep the surface of the heating element 20 flat, which is in favor of sleeve the heating element 20 on the sleeve 10. Of course, the temperature sensor 50 further needs to connect the power supply and the main board in the controlling circuit, configured for receiving and processing the temperature signals, so as to real-time monitor the heating element 20. The number of the temperature sensors 50 is equal to the number of the heating areas in the heating element 20, which may monitor each heating area in the heating element 20.

The present disclosure further relates to a low-temperature baked smoking system including the aforementioned vaporizer 400. The smoking set includes a power supply module configured for supplying power to the vaporizer 400. So the power supply module is electrically connected with the vaporizer 400 via a threaded connection.

## Claims

1. A low-temperature baked vaporizer, comprising:
a sleeve (10) for receiving vaporizable materials; and
a heating element (20) manufactured by metal materials and sleeved on the sleeve (10), and configured for heating the sleeve (10);
the heating element (20) comprises a plurality of through holes (221); and
the plurality of through holes (221) are arranged along a length direction of the heating element (20),
**characterised in that**
the plurality of through holes (221) are divided into a plurality sets (22) of through holes (221) and arranged along a breadth direction of the heating element (20), and wherein distances between adjacent through holes (221) in each set (22) decrease along the length direction; or
wherein the plurality of through holes (221) are divided into a plurality sets (22) of through holes (221) and arranged along the breadth direction of the heating element (20), wherein the through holes (221) in each set (22) are arrayed along the length direction and sizes of the through holes (221) in each set (22) decrease along the length direction.

2. The vaporizer according to claim 1, wherein:
the heating element (20) further comprises two electrode connecting parts (211, 212);
the two electrode connecting parts (211, 212) are arranged along the length direction; and
the plurality of through holes (221) are disposed between the two electrode connecting parts (211, 212).

3. The vaporizer according to any one of claims 1-2, wherein:
the heating element (20) comprises a cut configured for the heating element (20) to be easily sleeved on the sleeve (10); and
the cut is arranged on the heating element (20) along the length direction of the heating element (20).

4. A low-temperature baked smoking system, comprising:
the vaporizer according to any one claims 1-3; and
a power supply configured for supplying power to the vaporizer.

## Patentansprüche

1. Niedertemperatur-Backverdampfer, umfassend:
eine Hülse (10) zur Aufnahme von verdampfbaren Materialien; und
ein Heizelement (20), das aus metallischen Materialien hergestellt und auf die Hülse (10) aufgeschoben ist, und das zum Beheizen der Hülse (10) konfiguriert ist;
das Heizelement (20) eine Vielzahl von Durchgangslöchern (221) umfasst; und
die Vielzahl von Durchgangslöchern (221) entlang einer Längsrichtung des Heizelements (20) angeordnet sind,
**dadurch gekennzeichnet, dass**
die Vielzahl von Durchgangslöchern (221) in eine Vielzahl von Sätzen (22) von Durchgangslöchern (221) unterteilt sind und entlang einer Breitenrichtung des Heizelements (20) angeordnet sind, und wobei die Abstände zwischen benachbarten Durchgangslöchern (221) in jedem Satz (22) entlang der Längenrichtung abnehmen; oder
wobei die Vielzahl von Durchgangslöchern (221) in eine Vielzahl von Sätzen (22) von Durchgangslöchern (221) unterteilt und entlang der Breitenrichtung des Heizelements (20) angeordnet sind, wobei die Durchgangslöcher (221) in jedem Satz (22) entlang der Längsrichtung angeordnet sind und die Größen der Durchgangslöcher (221) in jedem Satz (22) entlang der Längsrichtung abnehmen.

2. Verdampfer nach Anspruch 1, wobei:
das Heizelement (20) ferner zwei Elektrodenanschlussteile (211, 212) umfasst;
die beiden Elektrodenanschlussteile (211, 212) entlang der Längsrichtung angeordnet sind; und
die Mehrzahl von Durchgangslöchern (221) zwischen den beiden Elektrodenanschlussteilen (211, 212) angeordnet sind.

3. Verdampfer nach einem der Ansprüche 1-2, wobei:
das Heizelement (20) einen Schnitt aufweist, der so gestaltet ist, dass das Heizelement (20) leicht auf die Hülse (10) aufgeschoben werden kann; und
der Einschnitt auf dem Heizelement (20) entlang der Längsrichtung des Heizelements (20) angeordnet ist.

4. Niedertemperatur-Rauchsystem, umfassend:
den Verdampfer nach einem der Ansprüche 1-3; und
eine Stromversorgung, die so konfiguriert ist, dass sie dem Verdampfer Energie zuführt.

## Revendications

1. Vaporisateur cuit à basse température, comprenant
un manchon (10) destiné à recevoir des matières à vaporiser ; et
un élément chauffant (20) fabriqué à partir de matériaux métalliques, monté sur le manchon (10) et configuré pour chauffer le manchon (10) ;
l'élément chauffant (20) comprend une pluralité de trous de passage (221) ; et
la pluralité de trous traversants (221) est disposée le long d'une direction longitudinale de l'élément chauffant (20),
**caractérisé par le fait que**
la pluralité de trous traversants (221) est divisée en plusieurs ensembles (22) de trous traversants (221) et disposée le long de la direction de la largeur de l'élément chauffant (20), et dans laquelle les distances entre les trous traversants adjacents (221) dans chaque ensemble (22) diminuent le long de la direction de la longueur ; ou
dans lequel la pluralité de trous traversants (221) est divisée en plusieurs ensembles (22) de trous traversants (221) et disposée le long de la direction de la largeur de l'élément chauffant (20), dans lequel les trous traversants (221) dans chaque ensemble (22) sont disposés le long de la direction de la longueur et les tailles des trous traversants (221) dans chaque ensemble (22) diminuent le long de la direction de la longueur.

2. Le vaporisateur selon la revendication 1, dans lequel :
l'élément chauffant (20) comprend en outre deux pièces de connexion d'électrode (211, 212) ;
les deux parties de connexion d'électrode (211, 212) sont disposées le long de la direction de la longueur ; et
la pluralité de trous de passage (221) est disposée entre les deux parties de connexion d'électrode (211, 212).

3. Le vaporisateur selon l'une des revendications 1-2, dans lequel :
l'élément chauffant (20) comprend une découpe configurée pour que l'élément chauffant (20) soit facilement manchonné sur le manchon (10) ; et
la découpe est disposée sur l'élément chauffant (20) dans le sens de la longueur de l'élément chauffant (20).

4. Système de fumage à basse température, comprenant
le vaporisateur selon l'une des revendications 1 à 3 ; et
une alimentation électrique configurée pour alimenter le vaporisateur.
